# FASCICULE DE BREVET EUROPEEN

(11) **EP 1 311 472 B1**
(45) Date de publication et mention de la délivrance du brevet: **20.10.2004**
(21) Numéro de dépôt: 01963050.8
(22) Date de dépôt: 02.08.2001
(51) Int. Cl.: C07C 239/20, C07F 9/40

(54) **PROCEDE DE PREPARATION D'ALCOXYAMINES A PARTIR DE NITROXYDES**
VERFAHREN ZUR HERSTELLUNG VON ALKOXYAMINEN AUS NITROXYLE
METHOD FOR PREPARING ALKOXYAMINES FROM NITROXIDES

(30) Priorité: 04.08.2000 FR 0010344
(43) Date de publication de la demande: 21.05.2003
(73) Titulaire: Atofina, 92800 Puteaux (FR)
(72) Inventeur: COUTURIER, Jean-Luc, F-69006 Lyon (FR); GUERRET, Olivier, 64230 Mazerolles (FR)
(86) Numéro de dépôt international: PCT/FR2001/002526
(87) Numéro de publication internationale: WO 2002/012149

(56) Documents cités:
- WO-A-98/40415

## Description

La présente invention a pour objet un procédé de préparation d'hydroxylamines α,β,β-trisubstituées, ci-après désignées par alcoxyamines, obtenues à partir de nitroxydes, utilisables notamment comme amorceurs des polymérisations radicalaires. L'utilisation des alcoxyamines telles que celles dérivées du (2,2,6,6-tétraméthylpipéridinyl)-N-oxyde (TEMPO) dans la préparation des macromolécules a donné lieu à de nombreuses publications.

Ainsi, Hawker C.J. et coll. (Macromolécules 1996, 29, pages 5245-5254) ont montré que l'utilisation d'alcoxyamines dérivées du TEMPO telles que le (2',2',6',6'-tétraméthyl-1'-pipéridinyloxy)méthylbenzène comme amorceurs de polymérisation radicalaire du styrène permettait de contrôler la polymérisation et d'accéder à des polymères bien définis avec de bas indices de polydispersité et ils ont constaté que les vitesses de polymérisation étaient sensiblement équivalentes aux vitesses obtenues lorsqu'ils utilisaient des amorceurs classiques tels que l'AIBN ou le peroxyde de benzoyle en présence de TEMPO.

Les alcoxyamines peuvent être préparées selon des méthodes connues dans la littérature. La méthode la plus courante implique le couplage d'un radical carboné avec un radical nitroxyde.

Si on désigne par : 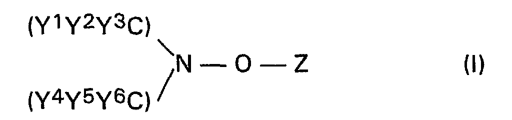 une alcoxyamine, Y¹, Y², Y³, Y⁴, Y⁵, Y⁶, Z étant définis plus loin, le radical carboné Z* peut être généré par différentes méthodes décrites dans la littérature : décomposition d'un composé azoïque, abstraction d'un atome d'hydrogène sur un substrat approprié, addition d'un radical sur une oléfine. Le radical Z* peut également être généré à partir d'un composé organométallique comme un organomagnésien Z-MgX tel que décrit par Hawker C.J. et coll. dans Macromolécules 1996, 29, 5245-5254 ou à partir d'un dérivé halogéné Z-X en présence d'un système organométallique comme CuX/bipyridine (X=Cl ou Br) selon une réaction de type ATRA (Atom Transfer Radical Addition) telle que décrit par Dorota Greszta et coll. dans Macromolecules 1996, 29, 7661-7670.

Une des méthodes les plus utilisées pour la préparation des alcoxyamines (I) est la méthode mettant en jeu la réaction ATRA.

Cette méthode consiste à transférer un atome ou un groupe d'atomes sur une autre molécule en présence d'un système organométallique CuX/bipyridine, en milieu solvant selon le schéma :

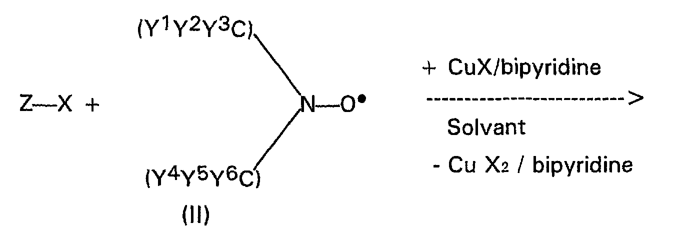

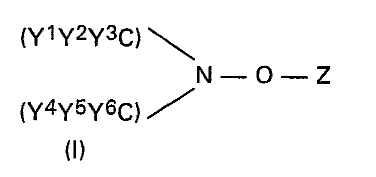

De préférence, X dans le système organométallique représente un atome de brome, de chlore ou d'iode.

Le mode opératoire généralement utilisé consiste à mettre en solution le système organométallique tel que CuBr/bipyridine dans un solvant organique de préférence aromatique tel que le benzène ou le toluène, puis à introduire dans la solution le composé ZX et le nitroxyde (II).

Cette façon d'opérer présente l'inconvénient majeur de nécessiter des durées de réactions longues, rédhibitoires pour une préparation industrielle d'alcoxyamines ou d'utiliser un large excès d'un des réactifs.

En outre, l'élimination du métal résiduel des produits obtenus est difficile, nécessitant des opérations coûteuses de purification telles que le passage des produits sur colonne de silice.

Ainsi, par exemple Matyjaszewski K. et coll, dans la demande de brevet internationale WO 98/40415 obtiennent le 1-(2,2,6,6-tétraméthylpipéridinyloxy)-1-phényléthane avec un rendement de 69 % après purification par chromatographie sur colonne en faisant réagir pendant 2 heures à 90°C le TEMPO et le (1-bromoéthyl)benzène selon un rapport molaire TEMPO/(1-bromoéthyl)benzène égal à 2 (soit un excès molaire de TEMPO égal à 100 %), en présence d'un système organométallique [4,4'-di(5-nonyl)2,2'-bipyridine/ Cu (OTf]₂/Cu°].

On a maintenant trouvé un procédé de préparation d'alcoxyamines de formule : 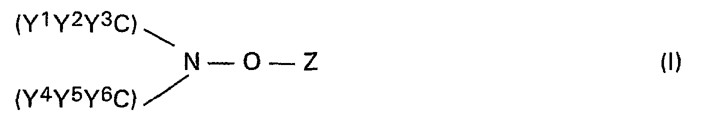 à partir de nitroxydes : 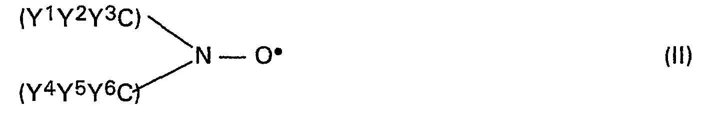 ledit procédé consistant à faire réagir ledit nitroxyde (II) avec un composé halogénocarboné ZX dans lequel X représente un atome de chlore, de brome ou d'iode, en présence d'un système organométallique MAₙ (L)_{y} (III) dans lequel :
M représente un métal de transition à un degré d'oxydation n tel qu'il puisse participer à une réaction rédox avec l'atome ou le groupe transférable,
A représente un atome d'halogène, un groupement carboxylate ou un groupement triflate,
L représente un ligand du métal M,
y est égal à 1, 2 ou 3,
n est égal à 1 ou 2, selon le schéma : 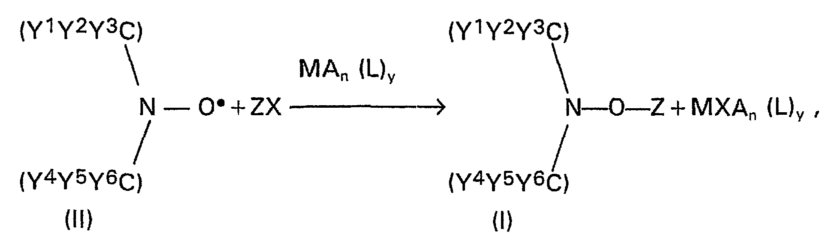
ledit procédé étant caractérisé en ce que l'on opère dans un milieu biphasique comprenant au moins un liquide ionique et un solvant organique non miscible avec ledit liquide ionique.

Le procédé selon l'invention consiste à effectuer les étapes suivantes :
a) on mélange sous agitation un sel métallique MAₙ, éventuellement un métal M au degré d'oxydation zéro, un ligand L, au moins un liquide ionique, un solvant organique, le composé halogénocarboné ZX et le nitroxyde (II) selon un rapport molaire ZX/nitroxyde (II) allant de 1 à 1,5 et, de préférence voisin de 1 ;
b) on maintient le milieu réactionnel sous agitation à une température comprise entre 20°C et 90°C et, de préférence, à une température allant de 20°C à 35°C jusqu'à disparition complète du nitroxyde (II) ; puis
c) on arrête l'agitation, on décante, on récupère la phase organique qui est éventuellement lavée à l'eau puis,
d) on isole l'alcoxyamine (I) de la phase organique par évaporation du solvant organique sous pression réduite, et
e) éventuellement, la phase liquide ionique est recyclée au moins une fois avec régénération de l'espèce active du métal M(Mⁿ).

Selon la présente invention, le nombre de recyclages de la phase liquide ionique n'est pas limitatif. De préférence, on recyclera la phase liquide ionique un nombre de fois allant de 1 à 10.

Selon la présente invention, on désigne par liquides ioniques des sels organiques liquides aux températures de réaction.

A titre d'illustration de sels organiques utilisables selon la présente invention, on citera les sels d'ammonium, de pyridinium, d'imidazolium, de triazolium, de guanidinium, de phosphonium ou de sulfonium. L'anion peut être entre autres un halogénure comme Cl⁻, Br⁻, I⁻ un halogénure d'étain comme SnCl₃⁻, un halogénure de germanium comme GeCl₃⁻, un halogénure de gallium comme GaCl₃⁻, un halogénure d'aluminium comme AlCl₄⁻, Al₂Cl₇⁻, Al₃Cl₁₀⁻, un halogénure de métal de transition comme CuCl₂⁻, un fluorure de bore, d'antimoine ou de phosphore comme BF₄⁻, SbF₆⁻ou PF₆⁻, un carboxylate comme CF₃CO₂⁻, un sulfonate comme CF₃SO₃⁻ ou FSO₃⁻, un amidure comme (CF₃SO₂)₂N⁻, un tétralkyl- ou tétraarylborure comme B(C₆F₅)₄⁻. Le cation et l'anion du sel ou du mélange de sels seront choisi de façon appropriée pour avoir un liquide à température de réaction. Eventuellement, le liquide ionique pourra contenir de l'eau. Selon la présente invention, on utilisera de préférence les sels de N,N'-dialkylimidazolium. Comme exemple de tels sels, on citera :
- le chlorure de 1-butyl-3-méthylimidazolium,
- le chlorure de 1-propyl-3-méthylimidazolium,
- le bromure de 1-propyl-3-méthylimidazolium.

Le solvant organique est choisi de façon à avoir un système biphasique avec le (ou les) liquide(s) ionique(s). De préférence, le solvant organique sera choisi parmi les hydrocarbures aliphatiques ou aromatiques, ou bien parmi les éthers. On utilisera tout particulièrement le toluène.

Selon la présente invention, M représente de préférence Cu(I), Fe(II), Ni(II) et, tout particulièrement Cu(I).

L'espèce active du métal M, ci-après Mⁿ, est générée à partir d'un sel métallique, de préférence un halogénure métallique MⁿXₙ. Elle peut également être générée in situ selon une réaction redox de type : 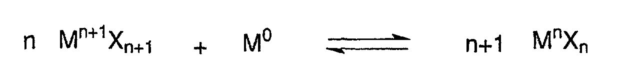 dans laquelle l'exposant du métal M représente le degré d'oxydation dudit métal, M^{o} représente le métal M au degré d'oxydation zéro et n = 1 ou 2.

Globalement, le ratio Mⁿ/RX doit être au moins égal à 1. L'halogénure métallique préféré est CuBr.

De préférence, A représente un halogène tel que Cl ou Br, un groupement carboxylate tel qu'acétate ou un groupement triflate et X représente un atome de chlore ou un atome de brome.

Selon la présente invention, le ligand L qui coordine le métal M peut contenir un ou plusieurs atomes d'azote, de phosphore, d'oxygène ou de soufre. Les ligands préférés sont les polyamines linéaires représentés par la formule générale (IV) : 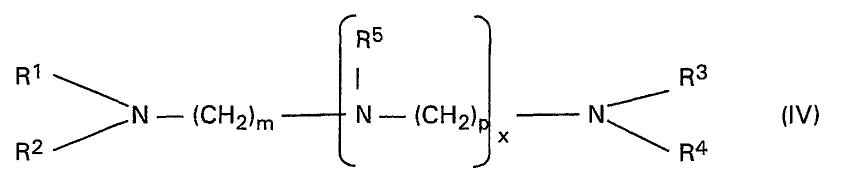 dans laquelle R¹, R², R³ et R⁴, identiques ou différents, représentent un atome d'hydrogène, un groupe alkyle, linéaire ou ramifié ayant un nombre d'atomes de carbone allant de 1 à 10 et, de préférence allant de 1 à 4, R⁵ représente un atome d'hydrogène, un groupe alkyle, linéaire ou ramifié, ayant un nombre d'atome de carbone allant de 1 à 10 et, de préférence, allant de 1 à 4, un reste 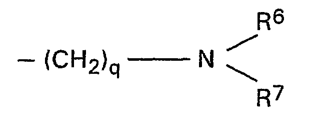 dans lequel R⁶ et R⁷ ont les mêmes significations que R⁵, ou bien encore au moins deux des radicaux R¹, R², R³, R⁴ et R⁵ peuvent être liés entre eux pour former un cycle ; m, p et q, identiques ou différents, représentent des nombres entiers allant de 1 à 4 et, de préférence, égaux à 2, x allant de 0 à 4.

A titre d'illustration de ligands L représentés par la formule (IV), on citera :
- la tris[2-(diméthylamino)éthyl]amine : 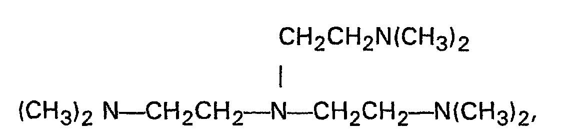
- la N, N, N', N', N" ― pentaméthyldiéthylènetriamine (PMDETA) : 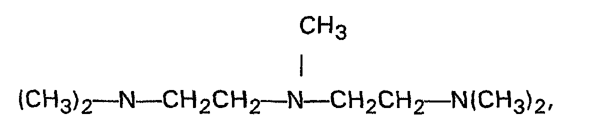
- la N,N,N',N'-tétraméthyléthylènediamine :

   (CH₃)₂―N―CH₂CH₂―N―CH₃)₂,
- la 1, 1, 4, 7, 10, 10-hexaméthyltriéthylènetétramine (HMTETA) : 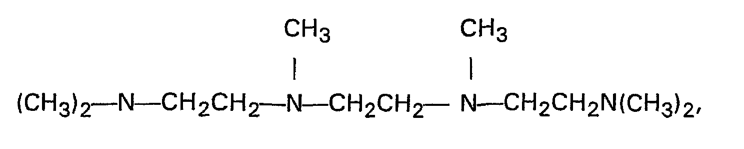
- la N,N-diméthyldipropylènetriamine (DMAPAPA) : 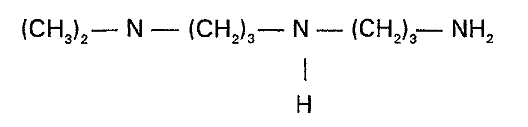
les polyamines cycliques telles que :
- le 1,4,7-triméthyl-1,4,7-triazacyclononane,
- le 1,5,9-triméthyl-1,5,9-triazacyclododécane,
- le 1,4,8,11-tétraméthyl-1,4,8,11-tétraazacyclotétradécane.

On utilisera de préférence la PMDETA et la DMAPAPA.

Le ligand L tel que défini précédemment est utilisé selon un rapport (molaire) L/Mⁿ allant de 0,2 à 4 et, de préférence allant de 0,4 à 2.

Le procédé selon l'invention consiste donc à mélanger sous forte agitation au moins un liquide ionique avec un sel métallique, un ligand L, le composé à atomes ou groupes transférables RX, le nitroxyde II et éventuellement le métal M au degré d'oxydation zéro et un solvant organique.

La réaction est conduite à une température comprise entre 20°C et 90°C, et de préférence voisine de la température ambiante. On opère sous atmosphère de gaz inerte tel que l'azote ou l'argon et de préférence à pression atmosphérique.

La fin de réaction peut être contrôlée par disparition des réactifs par des méthodes chromatographiques (CPG, HPLC, CCM). La réaction terminée, le mélange réactionnel est décanté. La phase organique est récupérée. La phase liquide ionique peut être éventuellement extraite avec le solvant organique de réaction. Les alcoxyamines (I) sont récupérées par des traitements classiques de la phase organique comme des lavages à l'eau suivis d'une évaporation du solvant. Les alcoxyamines (I) peuvent être caractérisées par analyse élémentaire, HPLC, IR et RMN.

Selon la présente invention, la phase liquide ionique peut être réutilisée pour une nouvelle réaction d'ATRA. La régénération du système organométallique peut être effectuée selon deux variantes.

Selon une première variante, la phase liquide ionique contenant le système organométallique peut être traitée avec le métal concerné au degré d'oxydation 0. La quantité de M⁰ rajoutée est comprise entre 0,5 et 2 équivalents par rapport au composé RX engagé initialement, de préférence voisine de 1 équivalent. La régénération de l'espèce active de métal Mⁿ peut être effectuée à une température comprise entre 20 ° C et 90°C, de préférence entre 20°C et 60°C, sur une durée comprise entre 0,5 et 3 heures. La phase liquide ionique peut ensuite être ainsi réutilisée pour une nouvelle réaction d'ATRA en rajoutant simplement le composé RX, le nitroxyde et le solvant organique et en procédant comme décrit précédemment. Ces cycles réaction/régénération du système organométallique peuvent être effectués plusieurs fois.

Selon une seconde variante, la régénération du système organométallique consiste à rajouter simultanément à la phase liquide ionique le composé RX, le nitroxyde, le solvant organique et le métal au degré d'oxydation 0 dans les proportions adéquates. La régénération s'effectue ainsi in situ et la réaction (d'ATRA) peut se produire comme décrit précédemment. Cette procédure peut également être répétée plusieurs fois.

Ainsi, un des avantages de la présente invention est que le système organométallique peut être régénéré sur plusieurs cycles sans rajout de sel métallique et de ligand. Ceci permet de réduire le coût du procédé en augmentant la productivité en alcoxyamine par rapport aux quantités de sel métallique et de ligand utilisées en système homogène.

En outre, le procédé selon l'invention présente l'avantage d'être réalisé avec des ligands disponibles dans le commerce. La réaction entre le nitroxyde (II) et le composé halogénocarboné ZX est rapide.

Le procédé selon l'invention permet d'obtenir des alcoxyamines exemptes quasiment de métal M. Les alcoxyamines obtenues selon le procédé de l'invention, présente une teneur en métal M inférieure à 10 ppm.

Les alcoxyamines sont ainsi obtenues avec des rendements élevés par un procédé facile à mettre en oeuvre et ne nécessitant pas d'opération coûteuse de purification.

Le procédé selon l'invention s'applique tout particulièrement à la préparation des alcoxyamines de formule : 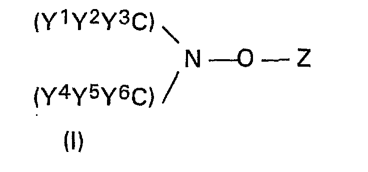 à partir de nitroxydes de formule : 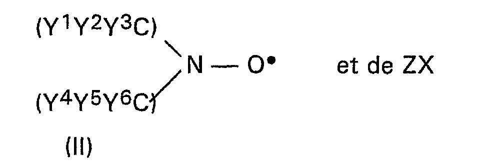 dans les formules desquelles les groupes Y¹ à Y⁶, identiques ou différents, représentent un atome d'hydrogène, un radical alkyle, linéaire ou ramifié, ayant un nombre d'atomes de carbone allant de 1 à 10, un radical cycloalkyle ayant un nombre d'atomes de carbone allant de 3 à 20, un atome d'halogène, un radical cyano, un radical phényle, un radical hydroxyalkyle ayant un nombre d'atomes de carbone allant de 1 à 4, un radical dialcoxyphosphonyle, diphénoxyphosphonyle, un radical alcoxycarbonyle, alcoxycarbonylalkyle, ou bien 2 ou plus des groupes Y¹ à Y⁶ peuvent être liés avec l'atome de carbone qui les porte pour former des structures cycliques, lesquelles peuvent comprendre une ou plusieurs fonctions extracycliques, choisis parmi : HO―, CH₃C(O)―, alkyl ―C(O)O ― ou le radical alkyle, linéaire ou ramifié, à un nombre d'atomes de carbone allant de 1 à 30, CH₃O―, H₂N―CH₃C(O)NH―, (CH₃)₂N― ; ou bien encore peuvent comprendre 1 ou plusieurs hétéroatomes extra- ou intracyliques tels que O, N ;

Z est un reste de formule 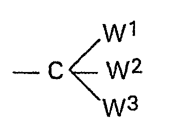 dans laquelle W¹, W² et W³, identiques ou différents, représentent un atome d'hydrogène, un radical alkyle, linéaire ou ramifié, ayant un nombre d'atomes de carbone allant de 1 à 10 , un radical phényle, un radical benzyle, un radical cyano, un radical cycloalkyle ayant un nombre d'atomes de carbone allant de 3 à 12 ; un radical -(CH₂)r C(O)OW⁴ dans lequel W⁴ représente un alkyle, linéaire ou ramifié, ayant un nombre d'atomes de carbone allant de 1 à 6 , r=0 à 6 ;
X représente un atome de chlore, de brome ou d'iode,

A titre d'illustration de nitroxydes (II) utilisables selon la présente invention, on citera :
- le 2,2,5,5 tétraméthyf-1-pyrrolidinyloxy (généralement commercialisé sous la marque PROXYL) ;
- le 3-carboxy-2,2,5,5-tétraméthyl-pyrrolidinyloxy (communément appelé 3-carboxy PROXYL) ;
- le 2,2,6,6-tétraméthyl-1-pipéridinyloxy (communément appelé TEMPO) ;
- le 4-hydroxy-2,2,6,6-tétraméthyl-1-pipéridinyloxy (communément appelé le 4-hydroxy-TEMPO) ;
- le 4-méthoxy-2,2,6,6-tétraméthyl-1-pipéridinyloxy (communément appelé le 4-méthoxy-TEMPO) ;
- le 4-oxo-2,2,6,6-tétraméthyl-1-pipéridinyloxy (communément appelé le 4-oxo-TEMPO) ;
- le 4-amino-2,2,6,6-tétraméthyl-1-pipéridinyloxy (communément appelé le 4-amino-TEMPO) ;
- le 4-acétamido-2,2,6,6-tétraméthyl-1-pipéridinyloxy (communément appelé le 4-acétamido-TEMPO) ;
- le 4-stéaryloxy-2,2,6,6-tétraméthyl-1-pipéridinyloxy (communément appelé le 4-stéaryloxy-TEMPO) ;
- le N-tertiobutyl-1-phényl-2-méthylpropyl nitroxyde,
- le N-(2-hydroxyméthylpropyl)-1-phényl-2-méthylpropylnitroxyde),
- le N-tertiobutyl-1-diéthylphosphono-2,2-diméthyl-propylnitroxyde,
- le N-tertiobutyl-1-dibenzylphosphono-2,2diméthylpropylnitroxyde,
- le N-tertiobutyl-1-di(2,2,2-trifluoroéthyl)phosphono-2,2diméthylpropylnitroxyde,
- le N-tertiobutyl-[(1-diéthylphosphono)-2-méthylpropyl]nitroxyde,
- le N-(1-méthyléthyl)-1-cyclohexyl-1-(diéthyl-phosphono)nitroxyde,
- le N-(1-phénylbenzyl)-[(1-diéthylphosphono)-1-méthyléthyl] nitroxyde :

- le N-phényl-1-diéthylphosphono-2,2-diméthylpropylnitroxyde,
- le N-phényl-1-diéthylphosphono-1-méthyléthylnitroxyde,
- le N-(1-phényl2-méthylpropyl)-1-diéthylphosphonméthyléthylnitroxyde,
- le bis-1-oxyl-2,2,6,6-tétraméthylpipéridine-4-yl)sébaçate commercialisé sous la marque "CXA 5415" par la Société CIBA SPEC. CHEM.

A titre d'illustration de composés ZX utilisables, on citera les composés de formule : C₆H₅CH(CH₃)Br, C₆H₅CH₂Br, (CH₃)₂C(CN)Br, CH₃OC(O)C(CH₃)₂Br, CH₃OC(O)CH(CH₃)Br, C₆F₁₃I.

Les alcoxyamines de formule (I) obtenues selon le procédé de la présente invention peuvent être utilisées pour la polymérisation et la copolymérisation de tout monomère présentant une double liaison carbone-carbone susceptible de polymériser par voie radicalaire. La polymérisation ou la copolymérisation est réalisée dans les conditions habituelles connues de l'homme du métier compte tenu du ou des monomères considérés. Les monomères considérés peuvent être un monomère vinylaromatique (styrène, styrènes substitués), un diène, un monomère acrylique ou méthacrylique. Le monomère peut également être le chlorure de vinyle, le difluorure de vinylidène ou l'acrylonitrile.

Les exemples suivants illustrent l'invention.

### EXEMPLES :

### REMARQUES GENERALES

Les essais ont été réalisés sous atmosphère de gaz inerte (argon ou azote) en employant des techniques de Schlenk (standard).

Le 1-bromoéthylbenzène et le N-tertobutyl-1-diéthylphosphono-2,2-diméthylpropylnitroxyde (DEPN) sont préalablement dégazés.

Le solvant utilisé est le toluène qui est préalablement distillé sous argon sur sodium-benzophénone.

Les ligands utilisés sont :
- la N,N,N',N',N"-pentaméthyldiéthyldiéthylènetriamine désignée ci-après par PMDETA,
- la bipyridine désignée ci-après par BIPY.
- Les alcoxyamines obtenues ont été caractérisées par RMN du ¹H, ¹³C et ³¹P, et par analyse élémentaire.

Les teneurs en cuivre résiduel ont été déterminées par la technique de spectroscopie d'émission atomique à plasma avec détection par spectrométrie de masse désignée ci-après ICP-MS (Inductively Coupled Plasma - Mass Spectrometry).

### Préparation des liquides ioniques utilisés

### 1/ Synthèse du bromure de 1-propyl-3-méthylimidazolium

Dans un réacteur en verre de 1 I, on charge 450 g de 1-bromopropane (3,66 mol). On chauffe à 70°C, puis sous agitation on coule goutte à goutte 200 g de 1-méthylimidazole (2,44 mol). On laisse réagir 1 h à 70°C. Après retour à température ambiante, on décante. La phase inférieure liquide ionique est récupérée, lavée au toluène (1x100 ml), puis évaporée sous vide. On obtient 475 g de bromure de 1-méthyl-3-propylimidazolium sous forme de liquide jaune. Le produit est utilisé tel quel sans purification supplémentaire.

### 2/ Synthèse du chlorure de 1-butyl-3-méthylimidazolium

Dans un réacteur en verre de 1 I, on charge 251,4 g de 1-chlorobutane (2,7 mol). On chauffe à 60°C, puis sous agitation on coule goutte à goutte 148,6 g de 1-méthylimidazole (1,8 mol). On laisse réagir 24 h à 80°C. Le mélange réactionnel est évaporé sous vide. On obtient 309 g de chlorure de 1-butyl-3-méthylimidazolium sous forme de solide jaunâtre (rendement=98%). Le produit est utilisé tel quel sans purification supplémentaire.

### EXEMPLE 1 (non conforme à l'invention)

### PREPARATION DU N-TERTIOBUTYL, N-1-DIETHYLPHOSPHONO-2,2-DIMETHYLPROPYL, O-1-PHENYLETHYLHYDROXYLAMINE :

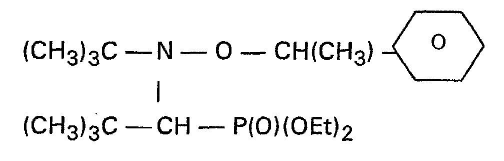

Dans un tube de Schlenk de 100 ml purgé à l'argon, on introduit 0,57 g de CuBr (4 mmol) et 1,25 g de BIPY (8 mmol) (rapport molaire BIPY/CuBr=2). On ajoute 0,74 g de (1-bromoéthyl)benzène (4 mmol) et 0,68 g de DEPN 86 % (2 mmol) dissous dans 9 ml de toluène anhydre. Sous agitation, on laisse réagir pendant 48 heures à température ambiante. Le mélange réactionnel est filtré sur célite. Le filtrat est lavé avec une solution aqueuse à 5 % de sulfate de cuivre, puis à l'eau. La phase organique est séchée sur sulfate de magnésium, puis le solvant est évaporé. On obtient une huile verdâtre contenant du cuivre qui est purifiée par chromatographie sur colonne de silice en utilisant un éluant pentane/éther 6/4. On obtient 0,75 g de N-tertiobutyl, N-1-diéthylphosphono-2,2-diméthylpropyl-O-1-phényléthylhydroxylamine (rendement=95 %) sous la forme de deux diastéréoisomères dans des proportions 64/36 déterminées sur le spectre ³¹P du mélange brut par intégration des signaux à 23,14 et 24,36 ppm (I/II = 64/36).

Les résultats analytiques sont donnés ci-après :

### Isomère I :

RMN ³¹P(CDCl₃) : δ 23,14

RMN ¹H(CDCl₃) : δ 0,88 (t,J_{H-H} =7,2Hz,3H) :

1,27 (m,21H) ; 1,55 (d, J_{H-H}=6,6Hz,3H) (s,9H) ; 3,40 (d,J_{H-P}=26Hz, 1H) ; 3,18-3,40 et 3,70-4,05 (m, 4H) ; 5,22 (q, J_{H-H}=6,6Hz, 1H) ; 7,24-7,47 (m,5H).

RMN ¹³C (CDCl₃) : δ 16,23 (2d,J_{C-P}=7Hz, CH₃CH₂), 21,18 (s,CH₃CH), 28,19 (s,CH₃-C-CH), 30,63 (d,J_{C-P}=7Hz, CH₃-CN), 35,33 (d,J_{C-P}=6Hz, C-CH-P), 58,58 (d, J_{C-P}=7,5Hz, C-CH₃), 61,4 (d,J_{C-P}=7Hz, CH₂-O), 70,06 (d, J_{C-P} = 138,5 Hz, CH-P), 78,36 (s, CH-O), 127,33 (s,CH ar), 127,81 (s,CH ar), 127,88 (s,CH ar), 143,31 (s, C ar).

Microanalyse (C₂₁H₃₇NO₄P) : % calculé C 63,12 ; H 9,59 ; N 3,51. % trouvé C 63,01 ; H 9,60 ; N 3,42.

### Isomère II :

RMN ³¹P (CDCl₃) : δ 24,36. RMN 1H (CDCl₃) : δ 0,82 (s,9H) ; 1,22 (s,9H) ; 1,29 (t, J_{H-H} 7,0Hz , 3H) ; 1,32 (t, J_{H-H} = 7,0Hz, 3H) ; 1,58 (d, J_{H-H}=6,7Hz, 3H) ; 3,32 (d, J_{H-P}=26,2Hz, 1H) ; 3,9-4,2 et 4,3-4,4 (m, 4H) ; 4,97 (q,J_{H-H}=6,8Hz, 1H) ; 7,17-7,3 (m, 5H).

RMN ¹³C (CDCl₃) : δ 16,24 (d,J_{C-P}=7,1Hz, CH₃CH₂), 16,71 (d, J_{C-P}=5,2Hz, CH₃CH₂), 24,00 (s, CH₃CH), 28,50 (s, CH₃-C-CH), 30,12 (d, J_{C-P}=5,7Hz, CH₃-C-N), 35,37 (d, J_{C-P}=5,8Hz, C-C_{H-P}), 58,80 (d, J_{C-P}=7,4Hz, CH₂-O), 61,10 (s, C-N), 61,56 (d, J_{C-P}=6Hz, CH₂-O), 69,84 (d, J_{C-P}=138,4Hz, CH-P), 85,23 (s, CH-O), 126,96 (s, CH ar), 127,08 (s, CH ar), 127,95 (s, CH ar), 145,36 (s, C ar).

Microanalyse (C₂₁H₃₇NO₄P) : % calculé C 63,12 ; H 9,59 ; N 3,51. % trouvé C 63,05 ; H 9,51 ; N 3,50.

### EXEMPLE 2 (conforme à l'invention)

### PREPARATION DU N-TERTIOBUTYL, N-1-DIETHYLPHOSPHONO-2,2-DIMETHYLPROPYL, O-1-PHENYLETHYLHYDROXYLAMINE EN PRESENCE DE BROMURE DE 1-METHYL-3-PROPYLIMIDAZOLIUM :

Dans un réacteur de 250 ml muni d'une agitation à turbine tournant à 500 tr/min purgé à l'argon, on charge 3,3 g de CuBr (23 mmol), 1,6 g de PMDETA (9 mmol), 1,46 g de Cu(O) (23 mmol) et 32 g de 1-méthyl-3-propylimidazolium. On rajoute 4,3 g de (1-bromoéthyl)benzène (23 mmol) et 5,56 g de DEPN 90% (17 mmol) dissous dans 60 g de toluène dégazé. On laisse réagir sous agitation à température ambiante. La disparition du DEPN est suivie par CCM. Après 24 heures, le mélange réactionnel est décanté. La phase organique est récupérée et la phase liquide ionique est extraite avec 60 g de toluène. Les phases organiques rassemblées sont lavées à l'eau (2x60 g). Le solvant est évaporé pour donner 6,6 g de N-tertiobutyl, N-1-diéthylphosphono-2,2-diméthylpropyl, O-1-phényléthylhydroxylamine sous forme d'huile incolore (rendement=97%, pureté=97%).

La teneur en cuivre résiduel est inférieure à 10 ppm.

La phase liquide ionique extraite est recyclée une première fois en lui ajoutant 1,46 g de Cu° (23 mmol), 4,3 g de (1-bromoéthyl)benzène (23 mmol), 5,56 g de DEPN 90% (17 mmol) dissous dans 60 g de toluène dégazé et on opère comme décrit comme précédemment. La phase liquide ionique extraite de ce premier recyclage est recyclée en lui ajoutant comme précédemment les mêmes réactifs et solvant en des quantités identiques. On effectue au total 4 recyclages. Les résultats sont résumés dans le tableau 1 suivant :

**TABLEAU 1**

| Essai | Chargement | T(°C) | t (h) | Rendement (%) |
|---|---|---|---|---|
| 1 | a | 20 | 24 | 97 |
| recyclage 1 | b | 20 | 24 | 95 |
| recyclage 2 | b | 20 | 24 | 95 |
| recyclage 3 | b | 20 | 24 | 97 |
| recyclage 4 | b | 20 | 24 | 93 |
| chargement a : CuBr=3,3 g ; PMDETA=1,6 g ; Cu°=1,46g ; (1-bromoéthyl)benzène=4,3 g ; DEPN 90%=5,6 g ; toluène=60 g ; 1-méthyl-3-propylimidazolium = 32 g chargement b : Cu°=1,46g ; (1-bromoéthyl)benzène=4,3 g ; DEPN 90%=5,6 g ; toluène=60 g | | | | |

Globalement, nous avons produit 32,4 g de N-tertiobutyl, N-1-diéthylphosphono-2,2-diméthylpropyl, O-1-phényléthylhydroxylamine (81 mmol) pour 3,3 g de CuBr (23 mmol), 1,6 g de PMDETA (9 mmol) et 7,3 g de Cu° (115 mmol).

### PREPARATION DU N-TERTIOBUTYL, N-1-DIETHYLPHOSPHONO-2,2-DIMETHYLPROPYL, O-1-PHENYLETHYLHYDROXYLAMINE EN PRESENCE DE BROMURE DE 1-PROPYL-3-METHYLIMIDAZOLIUM :

Dans cet exemple, la régénération de l'espèce active est effectuée préalablement à la réaction.

Dans un réacteur de 250 ml purgé à l'argon, on charge 3,3 g de CuBr (23 mmol), 4,0 g de PMDETA (23 mmol), 1,46 g de Cu° (23 mmol) et 32 g de 1-méthyl-3-propylimidazolium. On rajoute 4,3 g de (1-bromoéthyl)benzène (23 mmol) et 6,4 g de DEPN 92% (20 mmol) dissous dans 40 g de toluène dégazé. On laisse réagir 6 h sous agitation à 20°C. Le mélange réactionnel est décanté. La phase organique est récupérée, et la phase liquide ionique est extraite avec 20 g de toluène. Les phases organiques rassemblées sont lavées à l'eau (2x40 g). Le solvant est évaporé pour donner 7,6 g de N-tertiobutyl, N-1-diéthylphosphono-2,2-diméthylpropyl, O-1-phényléthylhydroxylamine sous forme d'huile incolore (rendement = 95%).

On rajoute ensuite à la phase liquide ionique extraite 1,46 g de Cu° et 20 g de toluène, puis on chauffe 3 h à 60°C. Une nouvelle réaction peut alors être effectuée en rajoutant 4,3 g de (1-bromoéthyl)benzène (23 mmol), 6,4 g de DEPN 92% (20 mmol) dissous dans 20 g de toluène. Selon cette procédure, 4 recyclages ont été effectués avec des durées réactionnelles telles qu'indiquées dans le tableau 2. Les résultats sont résumés dans le tableau 2 suivant :

**TABLEAU 2**

| Essai | Chargement | T (°C) | t (h) | Rendement (%) |
|---|---|---|---|---|
| 1 | a | 20 | 6 | 95 |
| recyclage 1 | b | 20 | 7 | 94 |
| recyclage 2 | b | 20 | 7 | 96 |
| recyclage 3 | b | 20 | 7 | 94 |
| recyclage 4 | b | 20 | 12 | 95 |
| chargement a : CuBr=3,3 g ; PMDETA=4,0 g ; Cu°=1,46g ; (1-bromoéthyl)benzène=4,3 g ; DEPN 92%=6,4 g ; toluène=40 g ; 1-méthyl-3-propylimidazolium = 32 g chargement b : Cu ° = 1,46g ; toluène=20g (régénération 3 h à 60°C) ; puis (1-bromoéthyl)benzène=4,3 g ; DEPN 92%=6,4 g ; toluène=20 g | | | | |

Globalement, nous avons produit 37,9 g de N-tertiobutyl, N-1-diéthylphosphono-2,2-diméthylpropyl, O-1-phényléthylhydroxylamine (95 mmol) pour 3,3 g de CuBr (23 mmol), 4 g de PMDETA (23 mmol) et 7,3 g de Cu° (115 mmol).

### Exemple 4 : Préparation du N-tertiobutyl, N-1-diéthylphosphono-2,2-diméthylpropyl, O-1-phényléthylhydroxylamine en présence de chlorure de 1-butyl-3-méthylimidazolium

Dans un réacteur de 250 ml purgé à l'argon, on charge 3,3 g de CuBr (23 mmol), 4,0 g de PMDETA (23 mmol), 1,46 g de Cu(O) (23 mmol), 32 g de 1-butyl-3-méthylimidazolium et 5 g d'eau. On rajoute 4,3 g de (1-bromoéthyl)benzène (23 mmol) et 5,4 g de DEPN 92% (17 mmol) dissous dans 40 g de toluène dégazé. On laisse réagir 3 h sous agitation à 35°C. Le mélange réactionnel est décanté. La phase organique est récupérée, et la phase liquide ionique est extraite avec 20 g de toluène. Les phases organiques rassemblées sont lavées à l'eau (2x40 g). Le solvant est évaporé pour donner 6,45 g de N-tertiobutyl, N-1-diéthylphosphono-2,2-diméthylpropyl, O-1-phényléthylhydroxylamine sous forme d'huile incolore (rendement=95%).

La régénération de l'espèce active est effectuée préalablement à la réaction selon les conditions ci-après.

On rajoute à la phase liquide ionique extraite 1,46 g de Cu° et 20 g de toluène, puis on chauffe 2 h à 35°C. Une nouvelle réaction peut alors être effectuée en rajoutant 4,3 g de (1-bromoéthyl)benzène (23 mmol), 5,4 g de DEPN 92% (17 mmol) dissous dans 20 g de toluène. Selon cette procédure, 9 recyclages ont été effectués avec des durées réactionnelles telles qu'indiquées dans le tableau 3. Les résultats sont résumés dans le tableau 3 suivant :

**TABLEAU 3**

| Essai | Chargement | T (°C) | t (h) | Rendement (%) |
|---|---|---|---|---|
| 1 | a | 35 | 3 | 95 |
| recyclage 1 | b | 35 | 3 | 94 |
| recyclage 2 | b | 35 | 3 | 97 |
| recyclage 3 | b | 35 | 3 | 95 |
| recyclage 4 | b | 35 | 3 | 93 |
| recyclage 5 | b | 35 | 1,5 | 92 |
| recyclage 6 | b | 35 | 3 | 96 |
| recyclage 7 | b | 35 | 3 | 97 |
| recyclage 8 | b | 35 | 3 | 95 |
| recyclage 9 | b | 35 | 3 | 95 |
| chargement a : CuBr=3,3 g ; PMDETA=4,0 g ; Cu° = 1,46g ; (1-bromoéthyl)benzène = 4,3 g ; DEPN 92%=5,4 g ; toluène = 40 g ; 1-butyl-3-méthylimidazolium = 32 g ; H₂O = 5 g chargement b : Cu°=1,46g ; toluène=20g (régénération 2 h à 35°C) ; puis (1-bromoéthyl)benzène=4,3 g ; DEPN 92%=5,4 g ; toluène=20 g | | | | |

Globalement, nous avons produit 64,5 g de N-tertiobutyl, N-1-diéthylphosphono-2,2-diméthylpropyl, O-1-phényléthylhydroxylamine (0,161 mol) pour 3,3 g de CuBr (0,023 mol), 4 g de PMDETA (0,023 mol) et 14,6 g de Cu° (0,230 mol).

## Revendications

1. Procédé de préparation d'alcoxyamines de formule : 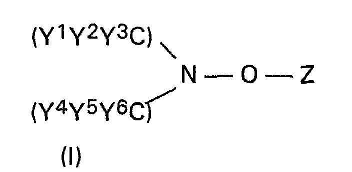 à partir de nitroxydes de formule : 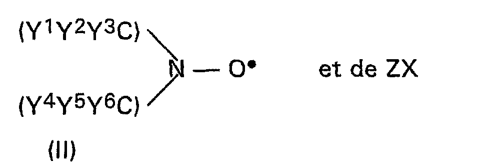 dans les formules desquelles les groupes Y¹ à Y⁶, identiques ou différents, représentent un atome d'hydrogène, un radical alkyle, linéaire ou ramifié, ayant un nombre d'atomes de carbone allant de 1 à 10, un radical cycloalkyle ayant un nombre d'atomes de carbone allant de 3 à 20, un atome d'halogène, un radical cyano, un radical phényle, un radical hydroxyalkyle ayant un nombre d'atomes de carbone allant de 1 à 4, un radical dialcoxyphosphonyle, diphénoxyphosphonyle, un radical alcoxycarbonyle, alcoxycarbonylalkyle, ou bien 2 ou plus des groupes Y¹ à Y⁶ peuvent être liés avec l'atome de carbone qui les porte pour former des structures cycliques, lesquelles peuvent comprendre une ou plusieurs fonctions extracycliques, choisis parmi : HO―, CH₃C(O)―, alkyl ―C(O)O ― ou le radical alkyle, linéaire ou ramifié, à un nombre d'atomes de carbone allant de 1 à 30, CH₃O―, H₂N―CH₃C(O)NH―, (CH₃)₂N― ; ou bien encore peuvent comprendre 1 ou plusieurs hétéroatomes extra- ou intracyliques tels que O, N ;
Z est un reste de formule 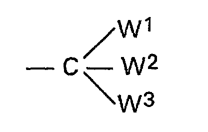 dans laquelle W¹, W² et W³, identiques ou différents, représentent un atome d'hydrogène, un radical alkyle, linéaire ou ramifié, ayant un nombre d'atomes de carbone allant de 1 à 10 , un radical phényle, un radical benzyle, un radical cyano, un radical cycloalkyle ayant un nombre d'atomes de carbone allant de 3 à 12 ; un radical -(CH₂)ᵣC(O)OW⁴ dans lequel W⁴ représente un alkyle, linéaire ou ramifié, ayant un nombre d'atomes de carbone allant de 1 à 6 , r=0 à 6 ;
X représente un atome de chlore, de brome ou d'iode,
ledit procédé consistant à faire réagir ledit nitroxyde (II) avec un composé halogénocarboné ZX, en présence d'un système organométallique MAₙ(L)_{y} (III) dans lequel:
M représente un métal de transition à un degré d'oxydation n tel qu'il puisse participer à une réaction redox avec l'atome ou le groupe transferrable,
A représente un atome d'halogène, un groupement carboxylate ou un groupement triflate,
L représente un ligand du métal M,
y est égal à 1, 2 ou 3,
n est égal à 1, ou 2,
selon le schéma : 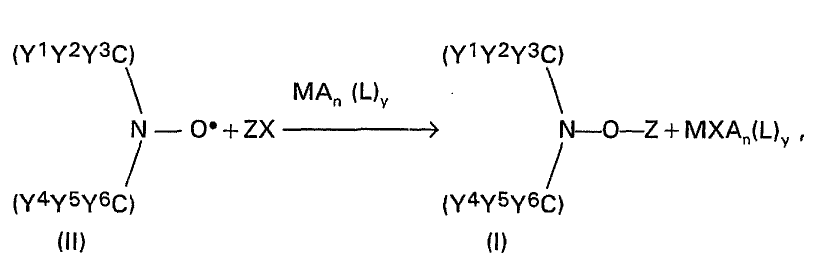 ledit procédé étant **caractérisé en ce que** l'on opère dans un milieu biphasique comprenant au moins un liquide ionique et un solvant organique non miscible avec ledit liquide ionique.

2. Procédé selon la revendication 1, **caractérisé en ce qu'**il consiste à effectuer les étapes suivantes :
a) on mélange sous agitation un sel métallique MAₙ, éventuellement un métal M au degré d'oxydation zéro, un ligand L, au moins un liquide ionique, un solvant organique, le composé halogéno carboné ZX et le nitroxyde (II) selon un rapport molaire ZX/nitroxyde (II) allant de 1 à 1,5 et, de préférence voisin de 1 ;
b) on maintient le milieu réactionnel sous agitation à une température comprise entre 20°C et 90°C et, de préférence, à une température allant de 20°C à 35°C jusqu'à disparition complète du nitroxyde (II) ; puis
c) on arrête l'agitation, on décante, on récupère la phase organique qui est éventuellement lavée à l'eau puis,
d) on isole l'alcoxyamine (I) de la phase organique par évaporation du solvant organique sous pression réduite, et
e) éventuellement la phase liquide ionique est recyclée au moins une fois et, de préférence un nombre de fois allant de 1 à 10, avec régénération de l'espèce active du métal M (Mⁿ).

3. Procédé selon l'une des revendications 1 ou 2, **caractérisé en ce que** le liquide ionique est choisi parmi les sels de N,N'dialkylimidazolium.

4. Procédé selon la revendication 3, **caractérisé en ce que** le liquide ionique est le chlorure de 1-butyl-3-méthylimidazolium, le chlorure de 1-propyl-3-méthylimidazolium ou le bromure de 1-propyl-3-méthylimidazolium.

5. Procédé selon la revendication 1, **caractérisé en ce que** le ligand L du métal M du système organométallique (III) est choisi parmi les composés représentés par la formule générale (IV) : 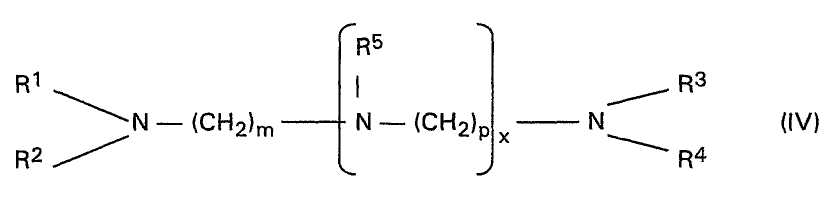 dans laquelle R¹, R², R³ et R⁴, identiques ou différents, représentent un atome d'hydrogène, un groupe alkyle, linéaire ou ramifié ayant un nombre d'atomes de carbone allant de 1 à 10 et, de préférence allant de 1 à 4, R⁵ représente un atome d'hydrogène, un groupe alkyle, linéaire ou ramifié, ayant un nombre d'atome de carbone allant de 1 à 10 et, de préférence, allant de 1 à 4, un reste 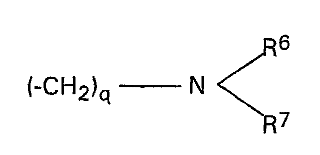 dans lequel R⁶ et R⁷ ont les mêmes significations que R⁵, ou bien encore au moins deux des radicaux R¹, R², R³, R⁴ et R⁵ peuvent être liés entre eux pour former un cycle ; m, p et q, identiques ou différents, représentent des nombres entiers allant de 1 à 4 et, de préférence, égaux à 2, x allant de 0 à 4.

6. Procédé selon la revendication 1 ou 2, **caractérisé par le fait que** M représente Cu(I), Fe(II), Ni(II) et, de préférence M représente Cu(I).

7. Procédé selon l'une des revendications 1 ou 2, **caractérisé par le fait que** A représente un atome de brome et X représente un atome de chlore ou un atome de brome.

8. Procédé selon l'une des revendications 1 à 4 **caractérisé en ce que** le rapport molaire ZX / nitroxyde (II) est voisin de 1.

9. Procédé selon l'une des revendications 1, 2, 6 ou 7, **caractérisé en ce que** le sel métallique MAₙ est un halogénure métallique MⁿXₙ dans la formule duquel l'exposant n du métal M représente le degré d'oxydation dudit métal et est égal à 1 ou 2.

10. Procédé selon la revendication 9, **caractérisé en ce que** l'halogénure métallique MⁿXₙ est CuBr.

11. Procédé selon l'une des revendications 1 à 7, **caractérisé en ce que** le rapport molaire L / espèce active Mⁿ va de 0,2 à 4.

12. Procédé selon la revendication 8, **caractérisé en ce que** le rapport molaire L / Mⁿ va de 0,4 à 2.

13. Procédé selon l'une des revendications 1 ou 2, **caractérisé en ce que** le solvant organique est un hydrocarbure aromatique.

14. Procédé selon la revendication 13, **caractérisé en ce que** l'hydrocarbure aromatique est le toluène.

15. Procédé selon l'une des revendications 1 à 14, **caractérisé en ce que** le ligand L est la N, N, N', N', N"-pentaméthyldiéthylènetriamine (PMDETA) ou la N,N-diméthyldipropylènetrimaine (DMAPAPA).

## Patentansprüche

1. Verfahren zur Herstellung von Alkoxyaminen der Formel: 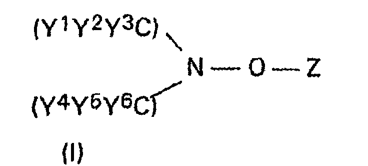 aus Nitroxiden der Formel: 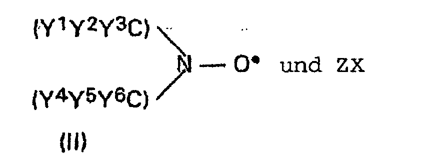 wobei in den Formeln die Gruppen Y¹ bis Y⁶ gleich oder verschieden sind und für ein Wasserstoffatom, einen linearen oder verzweigten Alkylrest mit 1 bis 10 Kohlenstoffatomen, einen Cycloalkylrest mit 3 bis 20 Kohlenstoffatomen, ein Halogenatom, einen Cyanorest, einen Phenylrest, einen Hydroxyalkylrest mit 1 bis 4 Kohlenstoffatomen, einen Dialkoxyphosphonylrest, einen Diphenoxyphosphonylrest, einen Alkoxycarbonylrest oder einen Alkoxycarbonylalkylrest stehen oder 2 oder mehr der Gruppen Y¹ bis Y⁶ mit dem Kohlenstoffatom, an das sie gebunden sind, zu cyclischen Strukturen, die eine oder mehrere exocyclische, unter HO-, CH₃C(O)-, Alkyl-C(O)O-, wobei der lineare oder verzweigte Alkylrest 1 bis 30 Kohlenstoffatome enthält, CH₃O-, H₂N-CH₃C(O)NH-, (CH₃)₂N- ausgewählte Funktionen oder auch ein oder mehrere exo- oder endocyclische Heteroatome, wie O oder N, enthalten können, verbunden sein können;
Z für einen Rest der Formel 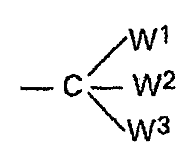 worin W¹, W² und W³ gleich oder verschieden sind und für ein Wasserstoffatom, einen linearen oder verzweigten Alkylrest mit 1 bis 10 Kohlenstoffatomen, einen Phenylrest, einen Benzylrest, einen Cyanorest, einen Cycloalkylrest mit 3 bis 12 Kohlenstoffatomen oder einen Rest -(CH₂)ᵣC(O)OW⁴, worin W⁴ lineares oder verzweigtes Alkyl mit 1 bis 6 Kohlenstoffatomen bedeutet und r gleich 0 bis 6 ist, stehen, steht und
X für ein Chlor-, Brom- oder Iodatom steht;
bei dem man das Nitroxid (II) in Gegenwart eines metallorganischen Systems MAₙ(L)_{y} (III), worin
M für ein Übergangsmetall in einer solchen Oxidationsstufe n, daß es an einer Redoxreaktion mit dem übertragbaren Atom oder der übertragbaren Gruppe teilnehmen kann, steht
A für ein Halogenatom, eine Carboxylatgruppe oder eine Triflatgruppe steht,
L für einen Liganden des Metalls M steht,
y gleich 1, 2 oder 3 ist und
n gleich 1 oder 2 ist,
mit einer Halogenkohlenstoffverbindung ZX gemäß dem Schema: 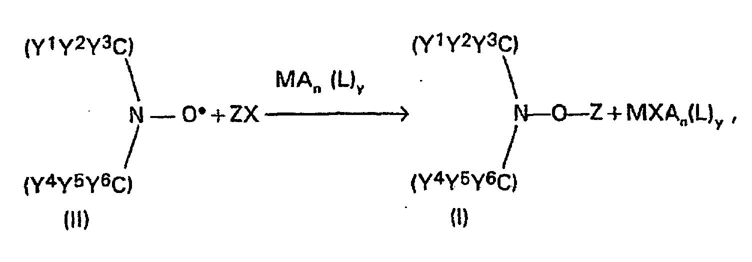 umsetzt,
**dadurch gekennzeichnet, daß** man in einem zweiphasigen Medium, das mindestens eine ionische Flüssigkeit und ein damit nicht mischbares organisches Lösungsmittel enthält, arbeitet.

2. Verfahren nach Anspruch 1, **dadurch gekennzeichnet, daß** man dabei so vorgeht, daß man:
a) ein Metallsalz MAₙ, gegebenenfalls ein Metall in der Oxidationsstufe null, einen Liganden L, mindestens eine ionische Flüssigkeit, ein organisches Lösungsmittel, die Halogenkohlenstoffverbindung ZX und das Nitroxid (II) in einem Molverhältnis von ZX zu Nitroxid (II) von 1 bis 1,5 und vorzugsweise ungefähr 1 unter Rühren vermischt;
b) das Reaktionsmedium bei einer Temperatur zwischen 20°C und 90°C und vorzugsweise einer Temperatur von 20 bis 35°C rührt, bis das Nitroxid (II) ganz verschwunden ist;
c) mit dem Rühren aufhört, absetzen läßt und die organische Phase zurückgewinnt und gegebenenfalls mit Wasser wäscht;
d) durch Abziehen des organischen Lösungsmittels unter vermindertem Druck das Alkoxyamin (I) aus der organischen Phase isoliert und
e) gegebenenfalls die ionische Flüssigkeit unter Regeneration der aktiven Spezies des Metalls M (Mⁿ) mindestens einmal und vorzugsweise 1-bis 10mal rezykliert.

3. Verfahren nach Anspruch 1 oder 2, **dadurch gekennzeichnet, daß** man die ionische Flüssigkeit unter N,N-Dialkylimidazoliumsalzen auswählt.

4. Verfahren nach Anspruch 3, **dadurch gekennzeichnet, daß** es sich bei der ionischen Flüssigkeit um 1-Butyl-3-methylimidazoliumchlorid, 1-Propyl-3-methylimidazoliumchlorid oder 1-Propyl-3-methylimidazoliumbromid handelt.

5. Verfahren nach Anspruch 1, **dadurch gekennzeichnet, daß** man den Liganden L des Metalls M des metallorganischen Systems (III) unter den Verbindungen der allgemeinen Formel (IV): 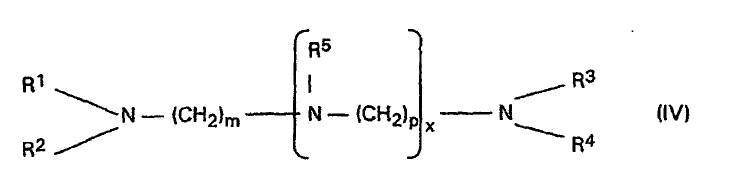 worin R¹, R², R³ und R⁴ gleich oder verschieden sind und für ein Wasserstoffatom oder eine lineare oder verzweigte Alkylgruppe mit 1 bis 10 Kohlenstoffatomen und vorzugsweise 1 bis 4 Kohlenstoffatomen stehen, R⁵ für ein Wasserstoffatom, eine lineare oder verzweigte Alkylgruppe mit 1 bis 10 Kohlenstoffatomen und vorzugsweise 1 bis 4 Kohlenstoffatomen oder einen Rest 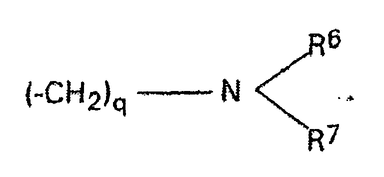 worin R⁶ und R⁷ die gleichen Bedeutungen wie R⁵ besitzen, steht oder auch mindestens zwei der Reste R¹, R², R³, R⁴ und R⁵ miteinander zu einem Ring verbunden sein können; m, p und q gleich oder verschieden sind und für ganze Zahlen von 1 bis 4 stehen und vorzugsweise gleich 2 sind und x im Bereich von 0 bis 4 liegt,
auswählt.

6. Verfahren nach Anspruch 1 oder 2, **dadurch gekennzeichnet, daß** M für Cu(I), Fe(II) oder Ni(II) und vorzugsweise für Cu(I) steht.

7. Verfahren nach Anspruch 1 oder 2, **dadurch gekennzeichnet, daß** A für ein Bromatom steht und X für ein Chloratom oder ein Bromatom steht.

8. Verfahren nach einem der Ansprüche 1 bis 4, **dadurch gekennzeichnet, daß** das Molverhältnis von ZX zu Nitroxid (II) ungefähr 1 beträgt.

9. Verfahren nach einem der Ansprüche 1, 2, 6 oder 7, **dadurch gekennzeichnet, daß** es sich bei dem Metallsalz MAₙ um ein Metallhalogenid MⁿXₙ, worin der Exponent n des Metalls M für die Oxidationsstufe des Metalls steht und gleich 1 oder 2 ist, handelt.

10. Verfahren nach Anspruch 9, **dadurch gekennzeichnet, daß** es sich bei dem Metallhalogenid MⁿXₙ um CuBr handelt.

11. Verfahren nach einem der Ansprüche 1 bis 7, **dadurch gekennzeichnet, daß** das Molverhältnis von L zu aktiver Spezies Mⁿ im Bereich von 0,2 bis 4 liegt.

12. Verfahren nach Anspruch 8, **dadurch gekennzeichnet, daß** das Molverhältnis von L zu Mⁿ im Bereich von 0,4 bis 2 liegt.

13. Verfahren nach Anspruch 1 oder 2, **dadurch gekennzeichnet, daß** es sich bei dem organischen Lösungsmittel um einen aromatischen Kohlenwasserstoff handelt.

14. Verfahren nach Anspruch 13, **dadurch gekennzeichnet, daß** es sich bei dem aromatischen Kohlenwasserstoff um Toluol handelt.

15. Verfahren nach einem der Ansprüche 1 bis 14, **dadurch gekennzeichnet, daß** es sich bei dem Liganden L um N,N,N',N',N"-Pentamethyldiethylentriamin (PMDETA) oder N,N-Dimethyldipropylentriamin (DMAPAPA) handelt.

## Claims

1. Process for preparing alkoxyamines of formula: 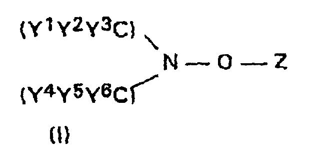 from nitroxides of formula: 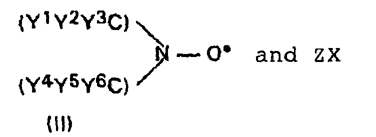 in which formulae the groups Y¹ to Y⁶, which may be identical or different, represent a hydrogen atom, a linear or branched alkyl radical containing a number of carbon atoms ranging from 1 to 10, a cycloalkyl radical containing a number of carbon atoms ranging from 3 to 20, a halogen atom, a cyano radical, a phenyl radical, a hydroxyalkyl radical containing a number of carbon atoms ranging from 1 to 4, a dialkoxyphosphonyl or diphenoxyphosphonyl radical, an alkoxycarbonyl or alkoxycarbonylalkyl radical, or alternatively 2 or more of the groups Y¹ to Y⁶ can be linked with the carbon atom which bears them to form cyclic structures, which can comprise one or more exocyclic functions chosen from: HO-, CH₃C(O)-, alkyl-C(O)O- or the linear or branched alkyl radical containing a number of carbon atoms ranging from 1 to 30, CH₃O-, H₂N-CH₃C(O)NH-, (CH₃)₂N-; or alternatively can comprise 1 or more exocyclic or endocyclic hetero atoms such as O or N;
Z is a residue of formula 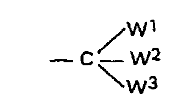 in which W¹, W² and W³, which may be identical or different, represent a hydrogen atom, a linear or branched alkyl radical containing a number of carbon atoms ranging from 1 to 10, a phenyl radical, a benzyl radical, a cyano radical, a cycloalkyl radical containing a number of carbon atoms ranging from 3 to 12; a radical -(CH₂)ᵣC(O)OW⁴ in which W⁴ represents a linear or branched alkyl containing a number of carbon atoms ranging from 1 to 6, r = 0 to 6;
X represents a chlorine, bromine or iodine atom,
the said process consisting in reacting the said nitroxide (II) with a halocarbon compound ZX, in the presence of an organometallic system MAₙ(L)_{y} (III) in which:
M represents a transition metal with an oxidation state n such that it can participate in a redox reaction with the transferable atom or group,
A represents a halogen atom, a carboxylate group or a triflate group,
L represents a ligand for the metal M,
y is equal to 1, 2 or 3,
n is equal to 1, or 2,
according to the scheme: 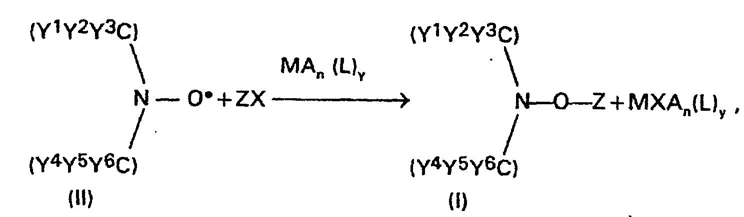 the said process being **characterized in that** the procedure is carried out in a biphasic medium comprising at least one ionic liquid and an organic solvent which is immiscible with the said ionic liquid.

2. Process according to Claim 1, **characterized in that** it consists in carrying out the following steps:
a) a metal salt MAₙ, optionally a metal M with an oxidation state zero, a ligand L, at least one ionic liquid, an organic solvent, the halo carbon compound ZX and the nitroxide (II) are mixed, with stirring, in a ZX/nitroxide (II) molar ratio ranging from 1 to 1.5, and preferably close to 1;
b) the reaction medium is kept stirring at a temperature of between 20°C and 90°C, and preferably at a temperature ranging from 20°C to 35°C, until the nitroxide (II) has completely disappeared; and then
c) the stirring is stopped, the mixture is separated by decantation, the organic phase is recovered and optionally washed with water, and then
d) the alkoxyamine (I) is isolated from the organic phase by evaporating the organic solvent under reduced pressure, and
e) optionally, the ionic liquid phase is recycled at least once, and preferably a number of times ranging from 1 to 10, with regeneration of the active species of the metal M(Mⁿ).

3. Process according to either of Claims 1 and 2, **characterized in that** the ionic liquid is chosen from N,N'-dialkylimidazolium salts.

4. Process according to Claim 3, **characterized in that** the ionic liquid is 1-butyl-3-methylimidazolium chloride, 1-propyl-3-methylimidazolium chloride or 1-propyl-3-methylimidazolium bromide.

5. Process according to Claim 1, **characterized in that** the ligand L for the metal M in the organometallic system (III) is chosen from the compounds represented by the general formula (IV): 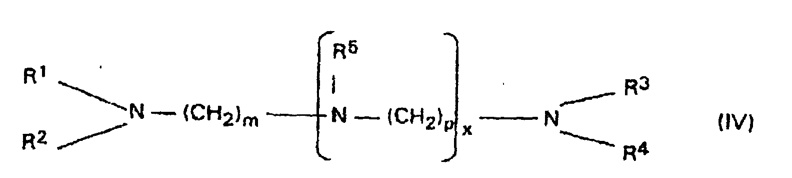 in which R¹, R², R³ and R⁴, which may be identical or different, represent a hydrogen atom, a linear or branched alkyl group containing a number of carbon atoms ranging from 1 to 10 and preferably ranging from 1 to 4, R⁵ represents a hydrogen atom, a linear or branched alkyl group containing a number of carbon atoms ranging from 1 to 10 and preferably ranging from 1 to 4, a residue 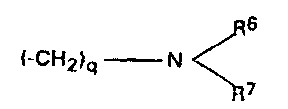 in which R⁶ and R⁷ have the same meanings as R⁵, or alternatively at least two of the radicals R¹, R², R³, R⁴ and R⁵ may be linked together to form a ring; m, p and q, which may be identical or different, represent integers ranging from 1 to 4 and preferably equal to 2, x ranging from 0 to 4.

6. Process according to Claim 1 or 2, **characterized in that** M represents Cu(I), Fe(II), Ni(II) and, preferably M represents Cu(I).

7. Process according to either of Claims 1 and 2, **characterized in that** A represents a bromine atom and X represents a chlorine atom or a bromine atom.

8. Process according to one of Claims 1 to 4, **characterized in that** the ZX/nitroxide (II) molar ratio is in the region of 1.

9. Process according to one of Claims 1, 2, 6 or 7, **characterized in that** the metal salt MAₙ is a metal halide (MⁿXₙ in the formula of which the superscript n of the metal M represents the oxidation state of the said metal and is equal to 1 or 2.

10. Process according to Claim 9, **characterized in that** the metal halide MⁿXₙ is CuBr.

11. Process according to one of Claims 1 to 7, **characterized in that** the molar ratio L/active species Mⁿ ranges from 0.2 to 4.

12. Process according to Claim 8, **characterized in that** the molar ratio L/Mⁿ ranges from 0.4 to 2.

13. Process according to either of Claims 1 and 2, **characterized in that** the organic solvent is an aromatic hydrocarbon.

14. Process according to Claim 13, **characterized in that** the aromatic hydrocarbon is toluene.

15. Process according to one of Claims 1 to 14, **characterized in that** the ligand L is N,N,N',N',N"-pentamethyldiethylenetriamine (PMDETA) or N,N-dimethyldipropylenetriamine (DMAPAPA).
